# DEMANDE DE BREVET EUROPEEN

(11) **EP 4 846 937 A1**
(43) Date de publication de la demande: **02.12.2026**
(21) Numéro de dépôt: 25179077.0
(22) Date de dépôt: 27.05.2025
(51) Int. Cl.: A61B 5/145, A61B 5/00

(54) **PROTEGE CAPTEUR**

(71) Demandeur: KittOFab SAS, 75012 Paris (FR)
(72) Inventeur: KIT, Alain Franck, 40230 Saint Geours de Maremme (FR); TIETTO, Mikäel Marc Alain, 40140 Soustons (FR)

(57) **Abrégé**

Le protège-capteur glycémique est un dispositif en silicone souple conçu pour sécuriser les capteurs glycémiques fixés sur la peau, souvent au bras. Il assure un maintien stable du capteur sans gêner son fonctionnement grâce à une ouverture centrale, tout en étant confortable et hypoallergénique. Réutilisable et facile à nettoyer, il protège le capteur des mouvements, frottements, transpiration, humidité et immersion dans l'eau, permettant ainsi à l'utilisateur de mener une vie active sans risque de décollage ou d'endommagement. Adaptable à différents modèles, il prolonge la durée de vie du capteur et améliore la liberté de mouvement au quotidien.

## Description

Le protège-capteur est un dispositif conçu pour sécuriser un capteur glycémique fixé sur la peau, généralement au niveau du bras .

Couramment utilisé dans la surveillance du diabète, le capteur glycémique est généralement maintenus par une simple couche adhésive. Or, cette fixation est souvent insuffisante face aux sollicitations du quotidien : mouvements brusques, frottements, transpiration, humidité, ou immersion dans l'eau.

Certains utilisateurs ont recours à des moyens artisanaux de maintien (bande adhésive, brassard), peu esthétiques et peu adaptés aux besoins médicaux. Aucun dispositif dédié, ergonomique, amovible et confortable n'est à ce jour largement disponible Le protège-capteur enveloppe le capteur de manière ajustée, assurant ainsi son maintien sans altérer son fonctionnement. Une ouverture centrale permet de laisser libre l'accès à la surface de lecture du capteur.

Sa forme ergonomique épouse les contours du capteur pour le stabiliser efficacement sans exercer de pression directe.

Il est fabriqué en silicone souple, flexible et doux ; une matière hypoallergénique et agréable au contact de la peau, évitant ainsi les irritations même sur peaux sensibles et offrant un confort optimal lors d'une utilisation prolongée.

Le protège-capteur est conçu pour être réutilisable et peut être nettoyé à l'eau claire. Grâce à sa conception, le protège-capteur permet à l'utilisateur de pratiquer des activités physiques, de se changer, de se doucher ou encore de nager, sans craindre de décoller ou d'endommager le capteur.

Il est adaptable à divers modèles de capteurs glycémiques.

Le protège-capteur contribue à la prolongation de la durée de vie du capteur en limitant les incidents liés à son arrachement et améliore la liberté de mouvement de l'utilisateur au quotidien.

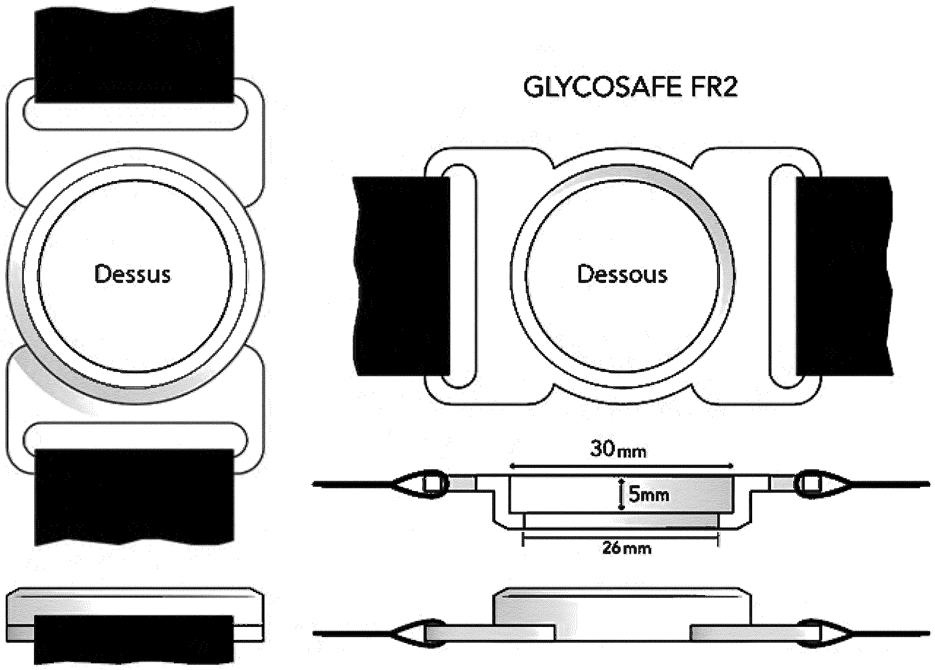

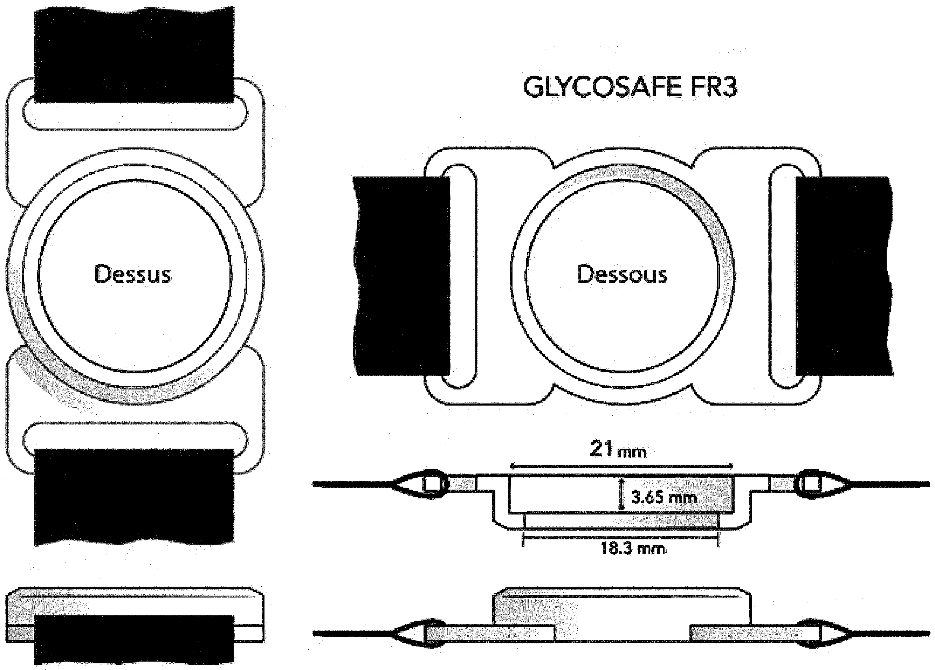

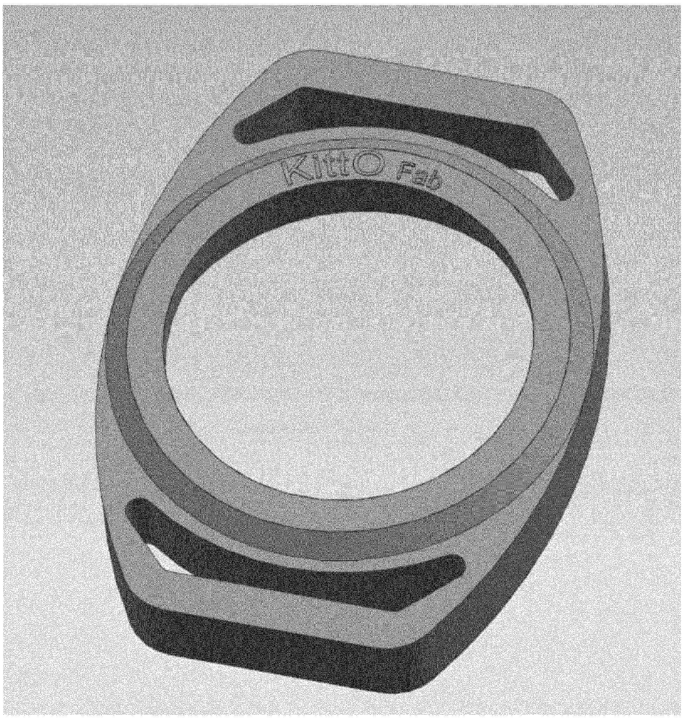

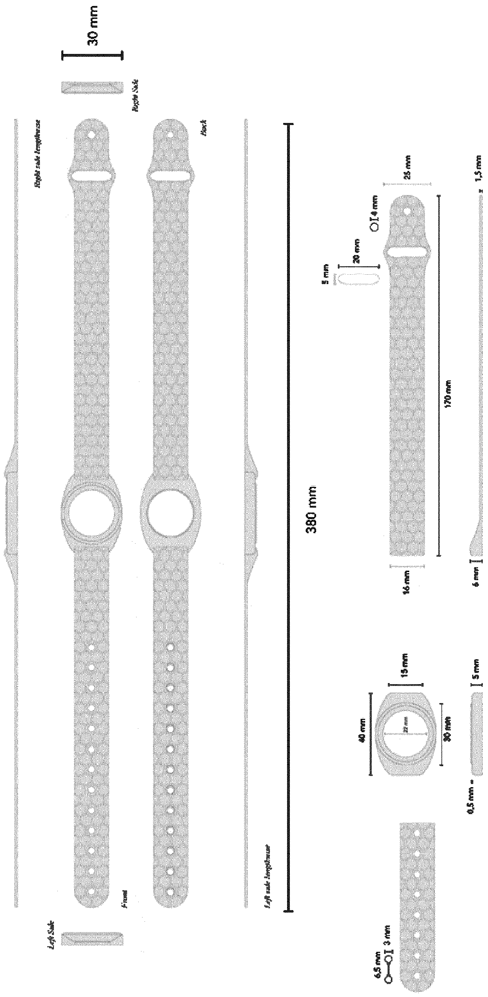

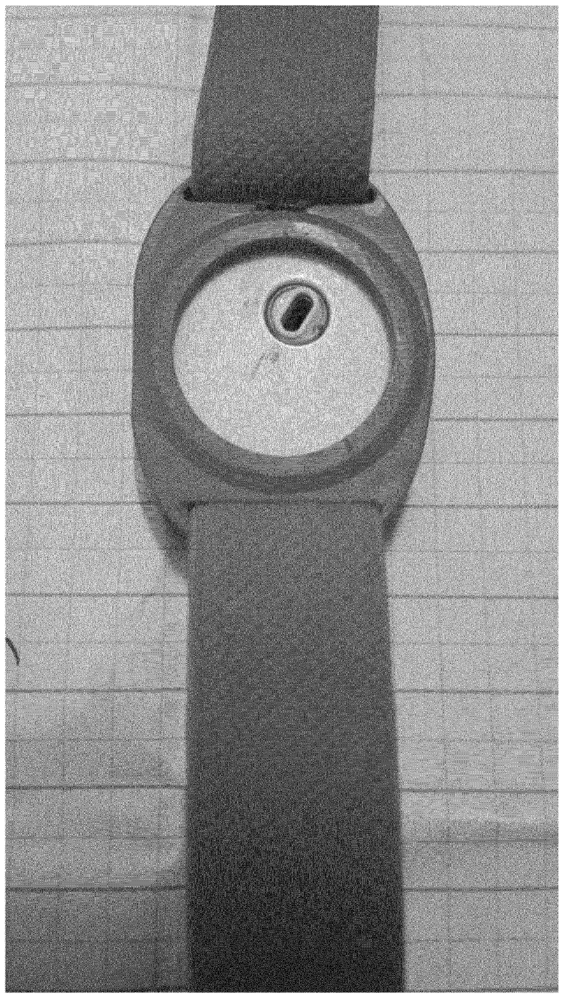

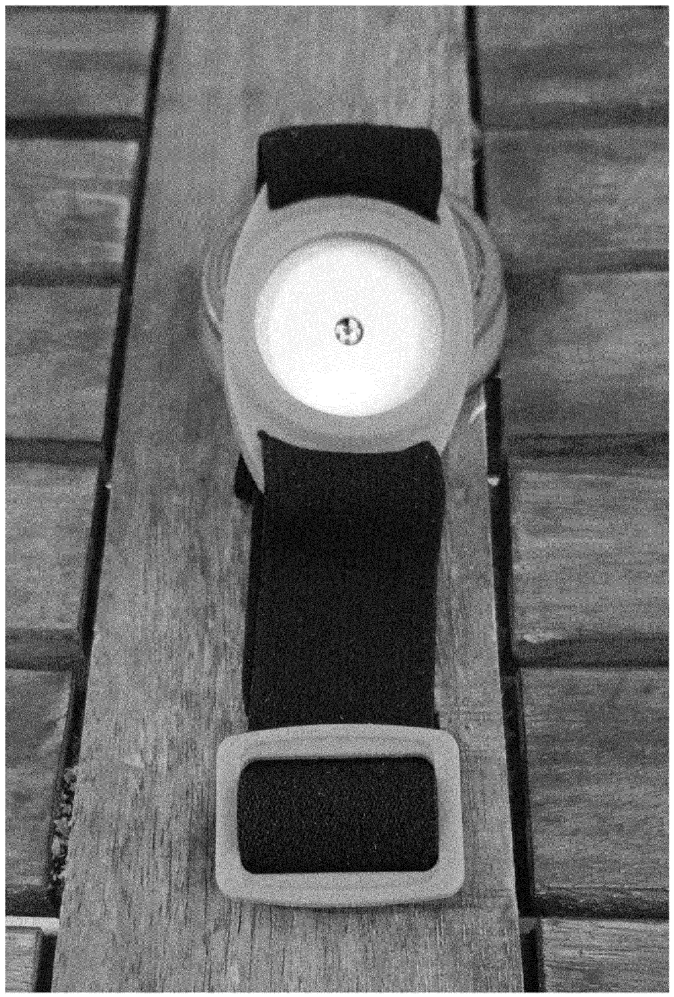

## Revendications

1. Dispositif de maintien pour capteur glycémique adhésif fixé sur la peau, **caractérisé en ce qu'**il comprend :
une structure souple en matériau silicone médical formant un contour apte à épouser la base dudit capteur,
une ouverture centrale laissant libre accès à la surface fonctionnelle dudit capteur,
une forme ergonomique adaptée à l'anatomie de l'utilisateur,
et un système de maintien permettant le port du dispositif en conditions normales d'activité (mouvement, contact avec l'eau, transpiration).

2. Dispositif selon la revendication 1, dans lequel le système de maintien est assuré par une bande élastique ou un brassard amovible.

3. Dispositif selon l'une quelconque des revendications précédentes, dans lequel la structure est réutilisable et lavable.

4. Dispositif selon l'une quelconque des revendications précédentes, conçu pour être compatible avec plusieurs marques ou formats de capteurs glycémiques.

## Revendications modifiées

### Revendications modifiées conformément à la règle 137(2) CBE.

1. Dispositif de maintien pour capteur glycémique adhésif fixé sur la peau, **caractérisé en ce qu'**il comprend :
une structure souple en matériau silicone médical formant un contour apte à épouser la base dudit capteur,
une ouverture centrale laissant libre accès à la surface fonctionnelle dudit capteur,
une forme ergonomique adaptée à l'anatomie de l'utilisateur,
et un système de maintien permettant le port du dispositif en conditions normales d'activité (mouvement, contact avec l'eau, transpiration).

2. Dispositif selon la revendication 1, dans lequel le système de maintien est assuré par une bande élastique ou un brassard amovible.

3. Dispositif selon l'une quelconque des revendications précédentes, dans lequel la structure est réutilisable et lavable.

4. Dispositif selon l'une quelconque des revendications précédentes, conçu pour être compatible avec plusieurs marques ou formats de capteurs glycémiques.

5. Dispositif selon l'une quelconque des revendications précédentes, **caractérisé en ce qu'**il comprend en outre une bague d'adaptation amovible montée de manière rotative au sein de l'ouverture centrale, ladite bague présentant une empreinte interne de forme complémentaire au profil du capteur glycémique pour en assurer le maintien, tout en permettant au bracelet de s'auto-ajuster à la morphologie du bras par rotation de ladite bague.
